# EUROPÄISCHE PATENTSCHRIFT

(11) **EP 1 062 926 B1**
(45) Veröffentlichungstag und Bekanntmachung des Hinweises auf die Patenterteilung: **25.02.2004**
(21) Anmeldenummer: 00113068.1
(22) Anmeldetag: 26.06.2000
(51) Int. Cl.: A61F 13/02

(54) **Pflaster mit vermindertem kalten Fluss**
Plaster with reduced cold flow
Sparadrap avec un fluage à froid réduit

(30) Priorität: 25.06.1999 DE 29911111 U
(43) Veröffentlichungstag der Anmeldung: 27.12.2000
(73) Patentinhaber: Novosis AG, 83714 Miesbach (DE)
(72) Erfinder: Fischer, Wilfried, Dr., 80807 München (DE); Kaffl, Hubert, 80807 München (DE); Huber, Petra, 80807 München (DE); Zimmermann, Clifton, 80807 München (DE)

(56) Entgegenhaltungen:
- US-A- 5 827 528

## Beschreibung

Die Erfindung betrifft ein Pflaster mit vermindertem kalten Fluß.

Unter "Pflaster" sollen im folgenden selbstklebende flächige Abdeckungen zur Aufbringung auf die menschliche Haut verstanden werden. Die Pflaster können hier ein- oder mehrschichtigen Aufbau haben und aus Folien, Geweben oder Vliesen in Kombination mit selbstklebenden Polymeren bestehen. Weiterhin können die Pflaster beliebige medizinische oder kosmetische Wirkstoffe enthalten oder auch wirkstofffrei sein. Die Anwendung kann dabei auf gesunder oder geschädigter Haut erfolgen.

Der Klebstoff der Pflaster hat in der Regel eine so ausreichende Fließfähigkeit, daß die Unebenheiten oder Rauhigkeiten der Haut umflossen werden können und sich dadurch eine gute Verbindung mit der Haut ergibt. Sehr "harte" Klebstoffe, die Glasübergangstemperaturen von > 10-20°C haben, kleben meistens auf Haut nicht gut. Ein gutes Fließverhalten des Klebstoffs führt aber andererseits dazu, daß an den Schnittkanten der fertigen Pflaster während der Lagerung oder während des Tragens eine geringe Klebstoffmenge austreten kann (sog. "kalter Fluß"), die z.B. während der Lagerung mit der Verpackung verkleben oder während der Applikationsdauer auf der Haut mit umgebender Kleidung oder anderen mit der Haut in Kontakt stehenden Gegenständen verkleben kann.

Derartige Pflaster sind auch Gegenstand der US-A-5,827,528. Darin werden, zwei Elastomere mit veschiedenen Eigenschaften gleichmäßig dispergiert. Derart beschaffene Pflaster weisen nach einiger Tragezeit einen kosmetisch sehr störenden "Schmutzrand" auf, der im allgemeinen durch sich an die an der Schnittkante austretenden Klebstoffmasse anhaftende Kleidungsfasern oder andere Partikel entsteht. Dabei kann der Schmutzrand direkt auf der Haut außerhalb des Pflasters oder unterhalb des äußeren Pflasterrandes auftreten.

Aufgabe der Erfindung ist, diese dem Stand der Technik anhaftenden Mängel zu beseitigen.

Diese Aufgabe wird erfindungsgemäß mit einem Pflaster mit vermindertem kalten Fluß mit den in Anspruch 1 angegebenen Merkmalen gelöst.

Weitere vorteilhafte Ausführungsformen sind Gegenstand der Unteransprüche.

### 2. Detaillierte Beschreibung der technischen Lösung

Die oben beschriebenen Nachteile des Standes der Technik werden durch die Kombination von mindestens zwei Klebstoffen vermieden, die räumlich getrennt voneinander auf das Pflaster aufgebracht werden.

Dabei wird um die Klebstoffschicht des eigentlichen Pflasters ("innere Klebstoffschicht" oder "Pflasterkern" bzw. kurz "Kern"), die z.B. einen medizinischen Wirkstoff enthält, herum eine zweite Klebstoffschicht ("äußere Klebstoffschicht" oder "Klebstoffring" bzw. kurz "Ring") aufgebracht, die den Klebstoff der inneren Schicht mechanisch am Ausfließen hindert. Es ist klar, daß dieser "Ring", solange er seine Funktion erfüllt, eine beliebige Form haben kann und zum Beispiel in der Aufsicht kreisförmig, oval oder auch quadratisch oder rechteckig, gegebenenfalls mit abgerundeten Kanten, ist. Die geeignete Form wählt der Fachmann nach Maßgabe der praktischen Gegebenheiten.

Im allgemeinen ist die innere Klebstoffschicht durch medizinische oder kosmetische Wirkstoffe oder andere Zusätze wie Harze oder Weichmacher so fließfähig und klebrig gemacht, daß sie eine gute und dauerhafte Verbindung mit der Haut ermöglicht.

Die äußere Klebstoffschicht hat dagegen eine stärkere Kohäsion und somit eine geringere Fließfähigkeit als die innere Klebstoffschicht, weil weichmachende Zusätze fehlen oder in einer geringeren Menge enthalten sind oder diese aus einem anderen, nicht so stark fließenden Polymer oder Polymergemisch besteht. Dadurch tritt am Schnittrand des Pflasters kein oder ein soweit verminderter kalter Fluß auf, daß ein Verkleben mit der Verpakkung oder das Auftreten eines Schmutzrandes auf der Haut bei längerer Tragezeit vermindert oder sogar vermieden werden kann. Gleichzeitig wird die fließfähige innere Klebstoffschicht mechanisch am Austreten gehindert.

Im einfachsten Fall werden nach dem Stand der Technik Pflaster hergestellt, indem eine Klebstofflösung oder -dispersion auf eine Folie, Gewebe oder Vlies durch Aufstreichen, Aufsprühen, Drucken o.ä. aufgebracht und diese Klebstoffschicht nach dem Trocknen mit einer zweiten Folie, Gewebe oder Vlies abgedeckt wird. Daran schließt sich die Formung der Pflaster durch Stanzen oder Schneiden und anschließend die Verpackung an.

Demgegenüber können die erfindungsgemäßen Pflaster mit vermindertem kalten Fluß beispielsweise folgendermaßen hergestellt werden.

### 2.1 Direktbeschichtung

### 2.1.1 Herstellung der Pflasterkerne

Eine silikonisierte Folie oder Papier (im folgenden nach der Funktion auch "Release-Liner" genannt) wird in einer geeigneten Beschichtungsmaschine mit dem Material des Pflasterkernes beschichtet und anschließend mit einer dünnen Abdeckfolie kaschiert. Mit einer Stanzmaschine werden die Pflasterkerne ausgestanzt, wobei die Stanze so einzujustieren ist, das die silikonisierte Folie nicht durchgestanzt wird (z.B. durch Anwendung des Kiss-cut-Verfahrens), d.h. die Kerne verbleiben auf der Folie. Anschließend wird die Folie aufgewickelt.

### 2.1.2 Herstellung der Pflaster mit Klebstoffkernen und -ringen

Die aufgewickelte Folie aus 2.1.1 mit den anhaftenden Pflasterkernen wird wiederum in einer Beschichtungsmaschine mit dem Material des Klebstoffrings beschichtet, so daß die Leerräume zwischen den Kernen mit dem Ringmaterial ausgefüllt werden. Die Beschichtung erfolgt so, daß die Abdeckfolie der Pflasterkerne nur minimal beschichtet wird. Nach dem gegebenenfalls notwendigen Trocknen soll die Klebstoffschicht zwischen den Kernen die gleiche Dicke aufweisen wie die Gesamtdicke der Pflasterkerne. Eventuell wird die Beschichtung so oft wiederholt bis die Dicke den gewünschten Wert erreicht hat. Anschließend wird mit einer zweiten Abdeckfolie kaschiert, so daß diese Folie nun sowohl das Material des Klebstoffringes als auch die Abdeckfolie des Pflasterkernes gleichmäßig bedeckt.

Aus dem Laminat werden nun in einem weiteren Stanzschritt die endgültigen Pflaster ausgestanzt. Dazu werden Stanzwerkzeuge benutzt, deren Durchmesser um das Doppelte der Breite des Kleberinges größer sind als die Pflasterkerne.

### 2.2 Separate Direktbeschichtung

Hierbei werden Pflasterkerne, Klebstoffringe und eine gemeinsame klebstoffbeschichtete Abdeckfolie getrennt hergestellt. Die Abdeckfolie verbindet später Pflasterkerne und Klebstoffringe.

### 2.2.1 Herstellung der Pflasterkerne

Die Herstellung erfolgt analog 2.1.1

### 2.2.2 Herstellung der Klebstoffringe

Eine silikonisierte Folie oder Papier wird in einer geeigneten Beschichtungsmaschine mit dem Material des Klebstoffringes beschichtet und anschließend mit einer dünnen Abdeckfolie kaschiert. Mit einer Stanzmaschine werden die Klebstoffringe ausgestanzt, wobei die Stanze so einzujustieren ist, das die silikonisierte Folie nicht durchgestanzt wird, d.h. die Ringe verbleiben auf der Folie. Anschließend wird die Folie aufgewickelt.

### 2.2.3 Herstellung der klebstoffbeschichteten Abdeckfolie

Eine dünne, nicht- oder einseitig silikonisierte Folie (z.B. Hostaphan MN 15) wird mit einer Schicht eines druckempfindlichen Haftklebers, z.B. einem Hotmelt-PSA oder einem UV-vernetzten PSA, mit einem Auftragsgewicht von ca. 1 - 20 g/m² beschichtet. Diese Beschichtung kann in einem separaten Coater oder direkt auf der Stanz- und Verpackunglinie vor der Montage von Kern und Ring erfolgen.

### 2.2.4 Herstellung der fertigen Pflaster

Einer geeigneten Stanzlinie für Pflaster wird die haftklebstoffbeschichtete Abdeckfolie aus 2.2.3 zugeführt. Von einer Abwicklung wird der z.B. die Kerne haltende Release-Liner so zugeführt, daß die Abdeckfolie der Kerne mit der Klebstoffschicht der Abdeckfolie aus 2.2.3 zusammengebracht und durch Druck (z.B. durch Anwendung einer Druckrolle) miteinander verbunden werden. Der Release-Liner der Kerne wird verworfen. Von einer zweiten Abwicklung wird dann der Release-Liner mit den Ringen so zugeführt, daß die Ringe konzentrisch um die Kerne aufgebracht werden.

Der Release-Liner der Ringe verbleibt auf dem Endprodukt. Nach der Laminierung werden die fertigen Pflaster z.B. mit einer Konturstanze ausgestanzt und anschließend verpackt.

### 2.3 Beschichtung mit Maskenfolien

Die eigentliche Pflasterklebstoffmasse, z.B. mit eingearbeiteten Wirkstoffen, wird mittels Druck-, Beschichtungs- oder Sprühverfahren auf eine Folie, Gewebe oder Vlies in der gewünschten Größe, vermindert um den umlaufenden späteren Klebstoffring aufgebracht und gegebenenfalls getrocknet oder erstarren gelassen.

Falls die Beschichtung durch Aufstreichen erfolgt, wird z.B. auf eine silikonisierte Folie (Release-Liner) vor dem Beschichten mit dem gewünschten Klebstoffgemisch durch Laminieren eine Maske aufgebracht.

### Herstellung der Lochmaskenfolie

Die Lochmaske wird hergestellt, indem auf eine beidseitig silikonisierte Papier- oder Polymerfolie (Intermediate-Liner 1) eine dünne Schicht eines druckempfindlichen Haftklebers aufgebracht wird, der auch für den späteren Klebstoffring verwendet wird. Die Auftragsstärke beträgt dabei etwa 1 bis 20 g/m². Nach dem Beschichten werden in einer geeigneten Stanz- oder Schneidevorrichtung Löcher der Größe und Form in die Folie gestanzt, die den Anforderungen an die späteren inneren Flächen (Pflasterkerne) der fertigen Pflaster entsprechen. Die Dicke der Folie inkl. der getrockneten Klebstoffschicht muß exakt der Naßschichtdicke der gewünschten Pflaster entsprechen, falls die spätere Beschichtung des Pflasterkerns mittels Lösemittelverfahren erfolgen soll, sonst der der Trockenschichtdicke.

### Herstellung der Pflasterkerne

Nach dem Beschichten wird die Lochmaskenfolie auf die silikonisierte Seite des Release-Liners aufkaschiert.

Nun wird das so entstandene Laminat weiter beschichtet, und zwar indem die in der Lochmaskenfolie ausgesparten Öffnungen mit dem Material der Pflasterkerne gefüllt werden. Nach dem eventuellen Trocknen der Füllungen wird das Laminat aufgerollt.

### Herstellung der Pflaster mit Klebstoffkernen und -ringen

In einer geeigneten Beschichtungsmaschine wird die Lochmaskenfolie vom Release-Liner der Kerne abgezogen und die Folie mit den nun auf der Silikonseite aufsitzenden Kernen erneut beschichtet, nämlich so, daß mit dem Material des Klebstoffringes die Zwischenräume zwischen den Kernen ausgefüllt werden. Die Dicke der Beschichtung richtet sich, unter Berücksichtigung des Schrumpffaktors der Schicht, nach der Dicke der Kerne. Nach der Trocknung oder Aushärtung des Ringmaterials soll sich möglichst die gleiche Schichtdicke wie die der Kerne ergeben.

Nach Verlassen des Coaters wird die zusammengesetzte Klebstoffschicht mit einer nicht silikonisierten Abdeckfolie kaschiert.

Aus dem Laminat werden nun in einem weiteren Stanzschritt die endgültigen Pflaster ausgestanzt. Dazu werden Stanzwerkzeuge benutzt, deren Durchmesser um das Doppelte der Breite des Klebstoffringes größer sind als die Pflasterkerne.

### 2.4 Herstellung durch Überkleben

In einer weiteren Ausführungsform werden zuerst die Pflasterkerne, wie in 2.2.1 beschrieben, hergestellt. Anschließend wird der Release-Liner mit den aufsitzenden Kernen vollständig mit einer klebstoffbeschichteten Abdeckfolie, wie in 2.2.3 beschrieben, kaschiert. Anschließend werden die endgültigen Pflaster ausgestanzt. Dazu werden Stanzwerkzeuge benutzt, deren Durchmesser um das Doppelte der Breite des Klebstoffringes größer sind als die Pflasterkerne.

## Patentansprüche

1. Flächiges selbstklebendes Pflaster mit mehrschichtigem Aufbau und vermindertem kalten Fluß, wobei der mehrschichtige Aufbau eine Abdeckung, eine Klebstoffschicht und einen auf der der Abdeckung gegenüberliegenden Seite der Klebstoffschicht befindlichen, temporär abdeckenden und abziehbaren Träger umfaßt, **dadurch gekennzeichnet, dass** die Klebstoffschicht einen Klebstoffkern aus einem fließfähigen Klebstoff und einen von diesem Klebstoffkern räumlich getrennten, den Klebstoffkern umgebenden Klebstoffring aus einem Klebstoff mit herabgesetzter Fließfähigkeit ohne kalten Fluß aufweist.

2. Pflaster nach Anspruch 1, **dadurch gekennzeichnet, dass** der Klebstoffkern einen medizinischen oder kosmetischen Wirkstoff enthält.

3. Pflaster nach Anspruch 1 oder 2, **dadurch gekennzeichnet, dass** die Abdeckung aus Kunststofffolie, Gewebe oder Vlies besteht.

4. Pflaster nach Anspruch 1, 2 oder 3, **dadurch gekennzeichnet, dass** der Träger aus Kunststofffolie, Papier oder einem Laminat daraus besteht.

5. Pflaster nach Anspruch 3 oder 4, **dadurch gekennzeichnet, dass** es sich bei der Kunststofffolie um Polyethylenterephthalat-, Polyethylen-, Polypropylen- oder Polyvinylchloridfolie handelt.

6. Pflaster nach Anspruch 4, **dadurch gekennzeichnet, dass** der Träger mit einer Trennmittelbeschichtung versehen ist.

7. Pflaster nach Anspruch 6, **dadurch gekennzeichnet, dass** die Trennmittelbeschichtung aus einer Silikon- oder Fluorsilikonschicht besteht.

## Claims

1. Flat self-adhering plaster having a multi-layer construction and reduced cold flow, wherein the multi-layer construction comprises a cover, a layer of adhesive and removable carrier, which acts as a temporary cover and which is present on the side of the layer of adhesive opposite the cover, **characterized in that** the layer of adhesive possesses a core of adhesive being a flowable adhesive and a ring of adhesive being an adhesive having reduced flowability without cold flow, spaced apart from said core and which surrounds said core.

2. Plaster according to claim 1, **characterized in that** the core of adhesive contains a pharmaceutical or cosmetic active agent.

3. Plaster according to claim 1 or 2, **characterized in that** the cover consists of a film of plastic, woven fabric or non-woven fabric.

4. Plaster according to claim 1, 2 or 3, **characterized in that** the carrier consists of a film of plastic, paper or a laminate thereof.

5. Plaster according to claim 3 or 4, **characterized in that** the film of plastic is a film of polyethylene terephthalate, polyethylene, polypropylene or polyvinyl chloride.

6. Plaster according to claim 4, **characterized in that** the carrier possesses a release coating.

7. Plaster according to claim 6, **characterized in that** the release coating consists of a silicone layer or fluorosilicone layer.

## Revendications

1. Pansement plat autocollant, doté d'une structure multicouche et coulant moins à froid, la structure multicouche comportant un revêtement, une couche d'adhésif et un support amovible constituant une couverture temporaire et disposé sur le côté de la couche d'adhésif opposé à celui où se trouve le revêtement, **caractérisé en ce que** la couche d'adhésif comporte un noyau d'adhésif constitué d'une colle fluide et un anneau d'adhésif qui entoure le noyau d'adhésif tout en étant spatialement séparé de lui et qui est constitué d'une colle à fluidité réduite qui ne coule pas à froid.

2. Pansement conforme à la revendication 1, **caractérisé en ce que** le noyau d'adhésif contient une substance active médicinale ou cosmétique.

3. Pansement conforme à la revendication 1 ou 2, **caractérisé en ce que** le revêtement est constitué d'une feuille de matière plastique ou d'un morceau de tissu ou de non-tissé.

4. Pansement conforme à la revendication 1, 2 ou 3, **caractérisé en ce que** le support est constitué d'une feuille de matière plastique ou d'un morceau de papier ou d'un stratifié de ces deux matières.

5. Pansement conforme à la revendication 3 ou 4, **caractérisé en ce que** la feuille de matière plastique est une feuille de poly(éthylène téréphtalate), de polyéthylène, de polypropylène ou de poly(chlorure de vinyle).

6. Pansement conforme à la revendication 4, **caractérisé en ce que** le support est doté d'une couche d'agent de séparation.

7. Pansement conforme à la revendication 6, **caractérisé en ce que** la couche d'agent de séparation consiste en une couche de silicone ou de fluorosilicone.
